# EUROPEAN PATENT SPECIFICATION

(11) **EP 3 342 453 B1**
(45) Date of publication and mention of the grant of the patent: **16.10.2019**
(21) Application number: 15902758.0
(22) Date of filing: 11.11.2015
(51) Int. Cl.: A61N 1/36, A61N 1/05

(54) **SMART PELVIC FLOOR MUSCLE REHABILITATION TRAINING DEVICE**
INTELLIGENTE VORRICHTUNG ZUM BECKENBODENMUSKULATURREHABILITATIONSTRAINING
DISPOSITIF D'ENTRAÎNEMENT INTELLIGENT POUR LA RÉÉDUCATION DES MUSCLES DU PLANCHER PELVIEN

(30) Priority: 28.08.2015 CN 201510541750
(43) Date of publication of application: 04.07.2018
(73) Proprietor: Nanjing Medlander Medical Technology Co., Ltd., Nanjing, Jiangsu 211112 (CN)
(72) Inventor: SHI, Zhihuai, Nanjing Jiangsu 211112 (CN); YANG, Ruijia, Nanjing Jiangsu 211112 (CN)
(74) Representative: Tiburzi, Andrea
(86) International application number: PCT/CN2015/094243
(87) International publication number: WO 2017/035951

(56) References cited:
- CN-A- 101 460 218
- CN-A- 101 460 218
- CN-A- 104 721 955
- CN-U- 202 822 489
- CN-U- 202 822 489
- CN-U- 203 576 501
- CN-U- 203 619 555
- US-A- 5 702 428
- US-B1- 6 289 245
- US-B2- 8 983 627

## Description

### Technical Field

The present invention relates to the technical field of medical instruments, and particularly to a smart pelvic floor muscle rehabilitation training device.

### Background Art

Electromyographic biofeedback training and neuromuscular electrical stimulation, belonging to physical therapies, are first-line therapeutic methods for treating pelvic floor dysfunctional diseases, wherein pelvic floor dysfunctional diseases (e.g. uroclepsia, organ prolapse, constipation, urinary retention, pelvic floor pain) are frequently-occurring diseases for postpartum women and menopausal women, and when a patient suffering from such disease sees a doctors in a hospital, a first-line therapeutic method comprises placing a vaginal electrode into the vagina of the patient, and a therapeutic apparatus performing pelvic floor electromyographic biofeedback training and neuromuscular electrical stimulation treatment by collecting a pelvic floor surface electromyographic signal or emitting an electric-impulse stimulation through the vaginal electrode. As such apparatus requires complicated operations and is expensive, individuals cannot afford it and can only be treated in the hospital; moreover, the treatment for each time lasts about 30 minutes, treatments for 10 to 15 times are required for one course of treatment, and the cure of the diseases generally requires 1 to 2 courses of treatment; the patient has to go to the hospital for each treatment and queue up for treatment, plenty of time is wasted due to routes to and from the hospital and the lining up, and patients having a job have to ask for leave for going to the hospital for treatment, and for this reason, many patients not having so severe symptoms abandon the treatment. CN101460218 discloses surgical procedures, kits and implants for alleviating human urinary and fecal incontinence; CN202822489 discloses an interactive pelvic floor muscle recovering device in the technical field of medical instruments, which comprises an electromyographic signal collecting circuit, a stimulating circuit, a power supply circuit, a reset circuit and a communication circuit; and US6289245 discloses an apparatus for medical treatment, which can reduce the load of signal processing and allows the patient to recognize the state of his/her muscular contraction more easily.

### Disclosure of the Invention

The invention is defined in claim 1. Further aspects and preferred embodiments are defined in the appended claims. Aspects, embodiments and examples of the present disclosure which do not fall under the scope of the appended claims do not form part of the invention and are merely provided for illustrative purposes. Furthermore, the methods presented in the present description are provided for illustrative purposes only and do not form part of the present invention.

Inventive object: In view of the shortages of the prior art, the present invention provides a smart pelvic floor muscle rehabilitation training device, wherein the device has low cost and a small volume, is easy to popularize, and during treatment, the device is directly placed in the vagina of a patient, and a smart mobile terminal is used for performing data processing, displaying and controlling, which facilitates treatment at home for the patient.

Technical solution: The smart pelvic floor muscle rehabilitation training device of the present invention comprises a portable vaginal electrode and a smart mobile terminal, wherein the vaginal electrode includes a pair of EMG (electromyographic) electrode plates and a pair of STIM (stimulation) electrode plates, with the pairs of electrode plates arranged on an outer wall of the vaginal electrode; and signal lines, an electromyographic amplifying circuit, an AD (Analog-to-Digital) sampling circuit, a master control MCU (microprogrammed control unit), an electrical stimulation circuit, a power-supply module and a wireless communication module that are packaged inside the vaginal electrode and in connection with the two pairs of electrode plates, wherein the power-supply module is in connection with all of the master control MCU, the electromyographic amplifying circuit, the AD sampling circuit, the electrical stimulation circuit and the wireless communication module; the EMG electrode plates are each used to collect an electromyographic signal from the pelvic floor muscle, the electromyographic signal is transmitted to the electromyographic amplifying circuit through the signal lines, the electromyographic amplifying circuit is in connection with the master control MCU through the AD sampling circuit, the master control MCU communicates with the smart mobile terminal through the wireless communication module, the smart mobile terminal is used to store training programs and feedback training results and send a stimulation instruction according to the training programs or results, the master control MCU is configured to control, based on the electromyographic signal or on the stimulation instruction received by the wireless communication module, the electrical stimulation circuit to emit a pulse current and the electrical stimulation circuit is in connection with the STIM electrode plates through the signal lines, and is configured to provide a neuromuscular electrical stimulation to the pelvic floor muscle.

To further complete the above-mentioned technical solution, the vaginal electrode has a front-end working area in the shape of a smooth water drop, an interior of a cavity structure, and a rear end emboied as a T-shaped handle area; two pairs of electrode plates are uniformly distributed in corresponding windows of a side wall of the cavity, a bar-type mainboard is provided in the middle of the cavity, with the signal lines, the electromyographic amplifying circuit, the AD sampling circuit, the master control MCU, the electrical stimulation circuit, the power-supply module and the wireless communication module being provided on the mainboard, wherein the front end of the mainboard is provided with a 4Pin single-row curved needle so as to be vertically connected with an upper cover plate provided with a matching 4Pin socket, and the upper cover plate is provided with 4 probes that are in connection with the 4Pin socket and are respectively in contact with and connected with the two pairs of electrode plates; and a keypad board is vertically connected with the tail end of the mainboard and is provided thereon with a button for switching on/off the device and an LED indicator light. Through the design of above-mentioned structures, the collection of the electromyographic signal, the processing of the electromyographic signal and the formation of the neuromuscular electrical stimulation are integrated on the vaginal electrode, and communication with the smart mobile terminal is realized through the wireless communication module, enabling patients to be treated at home, making the volume of the overall structure significantly reduced, making the structure easily portable, and realizing effective cost control and facilitating the popularization thereof.

Further, an inner wall of the vaginal electrode is provided with a boss structure, and the upper cover plate is fixed on the boss through a screw. The upper cover plate, as a connecting conductor for the electrode plates and the circuits on the mainboard, is an important component for assuring normal working of the vaginal electrode, wherein the upper cover plate is supported by the boss structure and tightly fastened by the screw, and stably connected with the inner wall of the vaginal electrode and the mainboard, ensuing that the electromyographic signal collected by the electrode plates is successfully transmitted to the mainboard.

Further, the T-shaped handle area is provided with a press area corresponding to the button and a display area corresponding to the LED indicator light.

Further, the wireless communication module is a Bluetooth module, and the smart mobile terminal is embodied as a smartphone or a tablet computer.

Further, a charging post is provided at a place where the T-shaped handle is connected with the working area, and the charging post has an inner side in connection with a charging interface of the power-supply module and an outer side in connection with a charging base. The arrangement of the charging post at the place where the T-shaped handle is connected with the working area does not affect the normal use by a patient, and the employment of the charging mode through the cooperation of a charging post with a charging base reduces the overall structure.

A pelvic floor muscle rehabilitation training method, which is not part of the invention, carried out by utilizing the above-mentioned smart pelvic floor muscle rehabilitation training device, comprises following steps:
(1) collecting, through the EMG electrode plates of the vaginal electrode placed in the vagina, an electromyographic signal produced when the pelvic floor muscle relaxes or contracts;
(2) transmitting the electromyographic signal through signal lines to an electromyographic amplifying circuit, and an AD sampling circuit converting the amplified electromyographic signal into a digital signal and inputting the same to the master control MCU;
(3) the master control MCU transmiting a result obtained after processing of the digital signal to a smart mobile terminal through a wireless communication module, and the smart mobile terminal feeding back the training result in a sound or screen display mode;
(4) the master control MCU generating a biofeedback signal according to the amplified electromyographic signal, an electrical stimulation circuit emitting a pulse current based on the biofeedback signal, and providing neuromuscular electrical stimulation to the pelvic floor muscle by the pulse current flowing through the signal lines and the STIM electrode plates; or the smart mobile terminal sending an electrical stimulation instruction to the master control MCU through the wireless communication module, the master control MCU emitting a pulse current through the electrical stimulation circuit, and provding neuromuscular electrical stimulation to the pelvic floor muscle by the pulse current flowing through the signal lines and the STIM electrode plates; and
(5) repeating Steps (2) to (4) to continuously collect electromyographic signals and update the biofeedback signals or stimulation instructions and form new neuromuscular electrical stimulation, and the smart mobile terminal performing real-time feedback on the results.

The screen display mode includes generation of a curve graph, a cartoon or a game.

Beneficial effects: In comparison with the prior art, the present invention has the following advantages. The smart pelvic floor rehabilitation training device of the present invention has a small volume, requires low cost, is powered by a 3.7 V polymer lithium battery, communicates with a smart mobile terminal using a low power Bluetooth module, and performs electromyographic biofeedback training and neuromuscular electrical stimulation by utilizing the powerful data processing function and display function of the smart mobile terminal. Through cost control, the product can be easily popularized and facilitate home therapy for patients; because of the small volume thereof, patients can conveniently carry around the product and can use it even when they go out; communication through a Bluetooth module is not restricted by a data cable, which facilitates the use; and it permits good privacy, better protecting the personal privacy of users.

### Brief Description of the Drawings

Fig. 1 is a schematic circuit diagram of the present invention.
Fig. 2 is a structural schematic diagram of the present invention.
Fig. 3 is a structural schematic diagram of a mainboard of the present invention.
Fig. 4 is a structural schematic diagram of an upper cover plate of the present invention.
Fig. 5 is a structural schematic diagram of a keypad board of the present invention.

### Detailed Description of the Embodiments

The technical solutions of the present invention will be described below in detail with reference to the accompanying drawings.

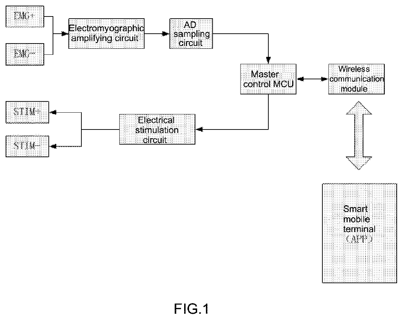

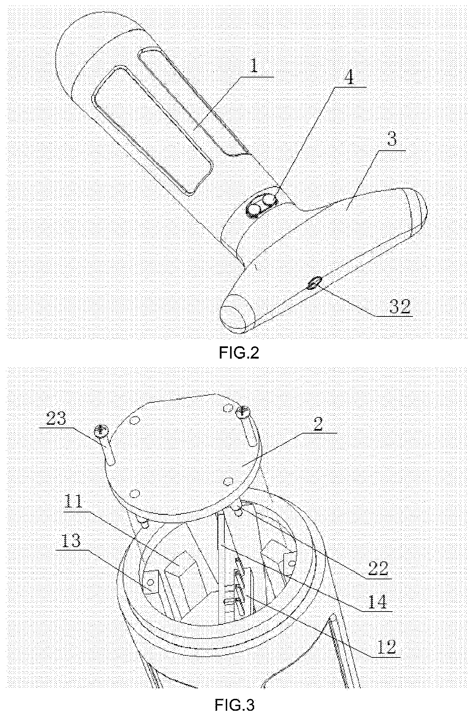

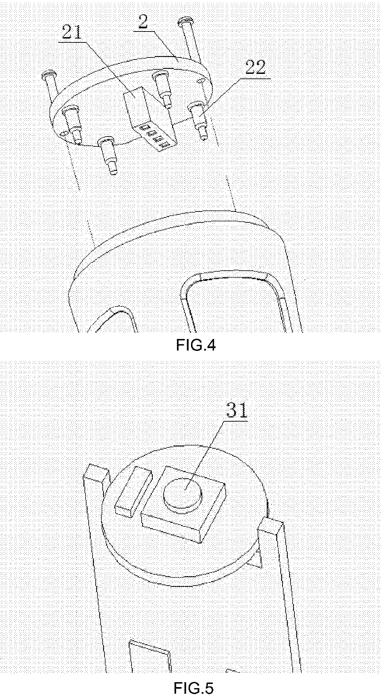

**Example 1:** An smart pelvic floor muscle rehabilitation training device as shown in Fig. 1 to Fig. 5 comprises a vaginal electrode and a smartphone, wherein the vaginal electrode has a front-end working area 1 in the shape of a smooth water drop, an interior of a cavity structure, and a rear end embodied as a T-shaped handle 3; four electrode plates 11 are arranged in corresponding windows of a side wall of the cavity, a bar-type mainboard 14 is provided in the middle of the cavity, with an EMG+ signal line, an EMG- signal line, an STIM+ signal line, an STIM- signal line, a 2.0 mm 4Pin single-row curved needle 12, an electromyographic amplifying circuit, an AD sampling circuit, a master control MCU, an electrical stimulation circuit, a power-supply module and a Bluetooth module being provided on the mainboard 14; a charging post 4 is provided at a place where the T-shaped handle 3 is connected with the working area 1, the charging post 4 has an inner side in connection with a charging interface of the power-supply module and an outer side in connection with a charging base, and the power-supply module is in connection with all of the master control MCU, the electromyographic amplifying circuit, the AD sampling circuit, the electrical stimulation circuit and the wireless communication module; the EMG+ signal line and the EMG-signal line are in connection with the electromyographic amplifying circuit, the electromyographic amplifying circuit is in connection with the master control MCU through the AD sampling circuit, the master control MCU is in connection with the electrical stimulation circuit, the electrical stimulation circuit is in connection with the STIM+ signal line and the STIM- signal line, the master control MCU is in connection with the smartphone through the Bluetooth module, and the EMG+ signal line, the EMG- signal line, the STIM+ signal line and the STIM- signal line are all in connection with the 2.0 mm 4Pin single-row curved needle 12; an inner wall of the vaginal electrode is provided with a structure in a form of boss 13, and an upper cover plate 2 is fixed on the boss 13 through a screw 23, wherein the upper cover plate 2 is provided with a 2.0 mm 4Pin socket 21 and four probes 22 in connection with the socket, with each probe 22 being in contact with one electrode plate 11; the upper cover plate 2 is vertically connected with the mainboard 14, and when connected, the 2.0 mm 4Pin single-row curved needle 12 on the mainboard 14 is exactly inserted into the 2.0 mm 4Pin socket 21 on the upper cover plate, so as to achieve the object of communication of the EMG+ signal line, the EMG- signal line, the STIM+ signal line and the STIM- signal line on the mainboard 14 with the four electrode plates 11, and after the communication, the four electrode plates respectively serve as an EMG+ electrode, an EMG- electrode, an STIM+ electrode and an STIM- electrode; a keypad board is vertically connected with the tail end of the mainboard 14 and is provided with a button 31 for switching on/off the device and an LED indicator light for indicating the on/off state and the working state of the device; and the T-shaped handle 3 is provided with a press area 32 corresponding to the button 31 and a display area for the LED indicator light, wherein the button 31 is pressed by exerting external force on the press area.

The EMG+ electrode and the EMG- electrode are used to collect a human body pelvic floor surface electromyographic signal, the STIM+ electrode and the STIM- electrode are used to perform neuromuscular electrical stimulation on the human body pelvic floor muscle, the EMG+ electrode and the EMG-electrode are in connection with the electromyographic amplifying circuit, the potential difference between the EMG+ electrode and the EMG- electrode is amplified by the electromyographic amplifying circuit by about 2,000 times, and then sent to the AD sampling circuit to convert the analog signal into a digital signal which is then sent to the master control MCU, the master control MCU communicates with the smartphone through the Bluetooth module, an APP software on the smartphone receives the pelvic floor surface electromyographic AD sampling data for data processing and analysis, and the result is fed back to the user in a mode of a curve graph, cartoon, sound and a game, so as to guide the user for performing pelvic floor electromyographic biofeedback training. The APP software on the smartphone may also send an electrical stimulation instruction to the master control MCU through the Bluetooth module, and the master control MCU controls the electrical stimulation circuit to emit a pulse current, which flows through the human body through the STIM+ electrode, and returns through the STIM- electrode, hereby providing neuromuscular electrical stimulation to the human body pelvic floor muscle. Alternatively, the electromyographic biofeedback function and the function of neuromuscular electrical stimulation may also be combined with each other to trigger neuromuscular electrical stimulation through the human body pelvic floor electromyographic signal, hereby achieving a better training effect, i.e., an electromyography-triggered electrical stimulation function.

A pelvic floor muscle rehabilitation training method, which is not part of the invention, utilizing the above-mentioned smart pelvic floor muscle rehabilitation training device comprises the following steps:
(1) collecting, through the EMG electrode plates of the vaginal electrode placed in the vagina, an electromyographic signal produced when the pelvic floor muscle relaxes or contracts;
(2) transmitting the electromyographic signal through the signal lines to the electromyographic amplifying circuit, and an AD sampling circuit converting the amplified electromyographic signal into a digital signal and inputting the same to the master control MCU;
(3) the master control MCU transmitting a result obtained after processing of the digital signal to the smart mobile terminal through the wireless communication module, and the smart mobile terminal feeding back the training result in a sound display mode or in a screen display mode by generating a curve graph, cartoon and a game;
(4) the master control MCU generating a biofeedback signal according to the amplified electromyographic signal, an electrical stimulation circuit emitting a pulse current based on the biofeedback signal, and providing a neuromuscular electrical stimulation to the pelvic floor muscle by the pulse current flowing through the signal lines and the STIM electrode plates; or the smart mobile terminal sending an electrical stimulation instruction to the master control MCU through the wireless communication module, the master control MCU emitting a pulse current through the electrical stimulation circuit, and providing a neuromuscular electrical stimulation to the pelvic floor muscle by the pulse current flowing through the signal lines and the STIM electrode plates; and
(5) repeating Steps (2) to (4) to continuously collect electromyographic signals and update the biofeedback signals or stimulation instructions, and form a new neuromuscular electrical stimulation, and the smart mobile terminal performing real-time feedback on the results.

## Claims

1. A smart pelvic floor muscle rehabilitation training device, comprising a portable vaginal electrode, signal lines, an electromyographic amplifying circuit, an Analog-to-Digital (AD) sampling circuit, a master control microprogrammed control unit (MCU), an electrical stimulation circuit, a power-supply module and a wireless communication module, wherein the vaginal electrode includes: a pair of electromyographic (EMG) electrode plates and a pair of stimulation (STIM) electrode plates, with the pair of EMG electrode plates and the pair of STIM electrode plates arranged on an outer wall of the vaginal electrode, the power-supply module is in connection with all of the master control MCU, the electromyographic amplifying circuit, the AD sampling circuit, the electrical stimulation circuit and the wireless communication module; the EMG electrode plates are each used to collect an electromyographic signal from a pelvic floor muscle, the electromyographic signal is transmitted to the electromyographic amplifying circuit through the signal lines, the electromyographic amplifying circuit is in connection with the master control MCU through the AD sampling circuit, **characterized in that** the smart pelvic floor muscle rehabilitation training device further comprises a smart mobile terminal, wherein the signal lines, the electromyographic amplifying circuit, the Analog-to-Digital (AD) sampling circuit, the master control microprogrammed control unit (MCU), the electrical stimulation circuit, the power-supply module and the wireless communication module are packaged inside the vaginal electrode and in connection with the two pairs of electrode plates, the master control MCU is configured to communicate with the smart mobile terminal through the wireless communication module, the smart mobile terminal is configured to store training programs and feedback training results and send an electrical stimulation instruction according to the training programs or results, the master control MCU is configured to control, based on the electromyographic signal or the stimulation instruction received by the wireless communication module, the electrical stimulation circuit to emit a pulse current, and the electrical stimulation circuit is in connection with the STIM electrode plates through the signal lines, and is configured to provide neuromuscular electrical stimulation to the pelvic floor muscle.

2. The smart pelvic floor muscle rehabilitation training device according to claim 1, **characterized in that** a front-end working area (1) of the vaginal electrode is in a shape of a smooth water drop, an interior of the vaginal electrode is of a cavity structure, and a rear end of the vaginal electrode is an area of a T-shaped handle; the two pairs of electrode plates are uniformly distributed in corresponding windows of a side wall of the cavity, a bar-type mainboard (14) is provided in middle of the cavity, the mainboard (14) is provided thereon with the signal lines, the electromyographic amplifying circuit, the AD sampling circuit, the master control MCU, the electrical stimulation circuit, the power-supply module and the wireless communication module, a front end of the mainboard (14) is provided with a 4Pin single-row curved needle (12), so as to be vertically connected with an upper cover plate (2) provided with a matching 4Pin socket (21), and the upper cover plate (2) is provided with 4 probes (22) that are in connection with the 4Pin socket (21), the 4 probes are respectively in contact with and connected with the two pairs of electrode plates; and a keypad board is vertically connected with a tail end of the mainboard (14) and the keypad board is provided thereon with a button (31) for switching on/off the device and an LED indicator light.

3. The smart pelvic floor muscle rehabilitation training device according to claim 2, **characterized in that** an inner wall of the vaginal electrode is provided with a structure in a form of boss (13), and the upper cover plate (2) is fixed on the boss (13) through a screw (23).

4. The smart pelvic floor muscle rehabilitation training device according to claim 2, **characterized in that** the T-shaped handle (3) is provided thereon with a press area (32) corresponding to the button (31) and a display area corresponding to the LED indicator light.

5. The smart pelvic floor muscle rehabilitation training device according to claim 1, **characterized in that** the wireless communication module is a Bluetooth module, and the smart mobile terminal is a smartphone or a tablet computer.

6. The smart pelvic floor muscle rehabilitation training device according to claim 1, **characterized in that** a charging post (4) is provided at a place where the T-shaped handle (3) is connected with the working area (1), with an inner side of the charging post (4) in connection with a charging interface of the power-supply module and an outer side of the charging post (4) in connection with a charging base.

## Patentansprüche

1. Intelligente Vorrichtung zum Beckenbodenmuskulaturrehabilitationstraining, umfassend eine tragbare Vaginalelektrode, Signalleitungen, eine elektromyografische Verstärkungsschaltung, eine Analog-Digital (AD) Abtastschaltung, eine mikroprogrammierte Master-Control Control-Unit (MCU), eine elektrische Simulationsschaltung, ein Stromversorgungsmodul und ein drahtloses Kommunikationsmodul, wobei die Vaginalelektrode beinhaltet: ein Paar von elektromyografischen (EMG) Elektrodenplatten und ein Paar von Simulationselektrodenplatten (STIM), wobei das Paar von EMG Elektrodenplatten und das Paar von STIM Elektrodenplatten an der Außenwand der Vaginalelektrode angeordnet sind, das Stromversorgungsmodul in Verbindung mit allen von der Master-Control MCU, der elektromyografischen Verstärkungsschaltung, der AD Abtastschaltung, der elektrischen Simulationsschaltung und dem drahtlosen Kommunikationsmodul verbunden ist; die EMG Elektrodenplatten jeweils verwendet werden, um ein elektromyografisches Signal von einem Beckenbodenmuskel zu sammeln, das elektromyografische Signal über die Signalleitungen zu der elektromyografischen Verstärkungsschaltung übertragen wird, die elektromyografische Verstärkungsschaltung durch die AD Abtastschaltung in Verbindung mit der Master-Control MCU ist, **dadurch gekennzeichnet, dass** die intelligente Vorrichtung zum Beckenbodenmuskulaturrehabilitationstraining weiter ein intelligentes mobiles Endgerät umfasst, wobei die Signalleitungen, die elektromyografische Verstärkungsschaltung, die Analog-Digital (AD) Abtastschaltung, die mikroprogrammierte Master-Control Control-Unit (MCU), die elektrische Simulationsschaltung, das Stromversorgungsmodul und das drahtlose Kommunikationsmodul in die Vaginalelektrode gepackt sind, und in Verbindung mit den beiden Paaren von Elektrodenplatten sind, die Master-Control MCU konfiguriert ist, um durch das drahtlose Kommunikationsmodul mit dem intelligenten mobilen Endgerät zu kommunizieren, das intelligente mobile Endgerät konfiguriert ist, Trainingsprogramme und Rückmelde-Trainingsergebnisse zu speichern, und eine elektrische Stimulierinstruktion an die Trainingsprogramme oder Ergebnisse zu senden, die Master-Control MCU konfiguriert ist, um basierend auf dem elektromyografischen Signal oder der Stimulierinstruktion, die von dem drahtlosen Kommunikationsmodul empfangen wird, die elektrische Simulationsschaltung zu steuern, einen Impulsstrom auszugeben, und die elektrische Simulationsschaltung durch die Signalleitungen in Verbindung mit den STIM Elektrodenplatten ist, und konfiguriert ist, um neuromuskuläre elektrische Stimulation für den Beckenbodenmuskel bereitzustellen.

2. Intelligente Vorrichtung zum Beckenbodenmuskulaturrehabilitationstraining nach Anspruch 1, **dadurch gekennzeichnet, dass** ein vorderer Arbeitsbereich (1) der Vaginalelektrode in einer Form eines glatten Wassertropfens ist, ein Inneres der Vaginalelektrode von einer Hohlraumstruktur ist, und ein hinteres Ende der Vaginalelektrode ein Bereich von einem T-förmigen Griff ist; die beiden Paare von Elektrodenplatten gleichförmig in entsprechenden Fenstern einer Seitenwand des Hohlraumes verteilt sind, eine stangenartige Hauptplatine (14) in der Mitte des Hohlraumes bereitgestellt ist, die Hauptplatine (14) darauf mit den Signalleitungen, der elektromyografischen Verstärkungsschaltung, der AD Abtastschaltung, der Master-Control MCU, der elektrischen Simulierschaltung, dem Stromversorgungsmodul und dem drahtlosen Kommunikationsmodul bereitgestellt ist, ein vorderes Ende der Hauptplatine (14) mit einer einreihigen gekrümmten 4Pin Nadel (12) bereitgestellt ist, um vertikal mit einer oberen Abdeckplatte (2) verbunden zu sein, die mit einer passenden 4Pin Steckdose (21) bereitgestellt ist, und die obere Abdeckplatte (2) mit 4 Sensoren (22) bereitgestellt ist, die in Verbindung mit der 4Pin Steckdose (21) sind, die 4 Sensoren jeweils mit den beiden Paaren von Elektrodenplatten in Kontakt und damit verbunden sind; und eine Tastenfeld-Platine vertikal mit einem Schwanzende der Hauptplatine (14) verbunden ist, und die Tastenfeld-Platine darauf mit einem Knopf (31) zum Ein-/Ausschalten der Vorrichtung und einem LED-Anzeigelicht bereitgestellt ist.

3. Intelligente Vorrichtung zum Beckenbodenmuskulaturrehabilitationstraining nach Anspruch 2, **dadurch gekennzeichnet, dass** eine Innenwand der Vaginalelektrode mit einer Struktur in Form eines Buckels (13) bereitgestellt ist und die obere Abdeckplatte (2) durch eine Schraube (23) auf dem Buckel (13) fixiert ist.

4. Intelligente Vorrichtung zum Beckenbodenmuskulaturrehabilitationstraining nach Anspruch 2, **dadurch gekennzeichnet, dass** der T-förmige Griff (3) darauf mit einem Drückbereich (32) entsprechend dem Knopf (31) und einem Anzeigebereich entsprechend dem LED-Anzeigelicht bereitgestellt ist.

5. Intelligente Vorrichtung zum Beckenbodenmuskulaturrehabilitationstraining nach Anspruch 1, **dadurch gekennzeichnet, dass** das drahtlose Kommunikationsmodul ein Bluetooth-Modul ist, und das intelligente mobile Endgerät ein Smartphone oder ein Tablet-Computer ist.

6. Intelligente Vorrichtung zum Beckenbodenmuskulaturrehabilitationstraining nach Anspruch 1, **dadurch gekennzeichnet, dass** eine Ladestation (4) an einem Platz bereitgestellt ist, wo der T-förmige Griff (3) mit dem Arbeitsbereich (1) verbunden ist, wobei eine Innenseite der Ladestation (4) in Verbindung mit einer Ladeschnittstelle des Stromversorgungsmoduls ist und eine Außenseite der Ladestation (4) in Verbindung mit einer Ladebasis ist.

## Revendications

1. Dispositif intelligent d'entraînement à la réadaptation des muscles du plancher pelvien, comprenant une électrode vaginale portative, des lignes de signal, un circuit d'amplification électromyographique, un circuit d'échantillonnage Analogique-Numérique (AD), une unité de commande microprogrammée (MCU) à commande maître, un circuit de stimulation électrique, un module d'alimentation en énergie et un module de communication sans fil, dans lequel l'électrode vaginale inclut : un couple de plaques d'électrodes électromyographiques (EMG) et un couple de plaques d'électrodes de stimulation (STIM), le couple de plaques d'électrodes EMG et le couple de plaques d'électrodes STIM étant agencés sur une paroi extérieure de l'électrode vaginale, le module d'alimentation en énergie est en connexion avec la totalité de la MCU à commande maître, du circuit d'amplification électromyographique, du circuit d'échantillonnage (AD), du circuit de stimulation électrique et du module de communication sans fil ; les plaques d'électrode EMG sont chacune utilisées pour recueillir un signal électromyographique provenant d'un muscle du plancher pelvien, le signal électromyographique est transmis au circuit d'amplification électromyographique par l'intermédiaire des lignes de signal, le circuit d'amplification électromyographique est en connexion avec la MCU à commande maître par l'intermédiaire du circuit d'échantillonnage AD, **caractérisé en ce que** le dispositif intelligent d'entraînement à la réadaptation des muscles du plancher pelvien comprend en outre un terminal mobile intelligent, dans lequel les lignes de signal, le circuit d'amplification électromyographique, le circuit d'échantillonnage Analogique-Numérique (AD), l'unité de commande microprogrammée (MCU) à commande maître, le circuit de stimulation électrique, le module d'alimentation en énergie et le module de communication sans fil sont incorporés à l'intérieur de l'électrode vaginale et en connexion avec les deux couples de plaques d'électrodes, la MCU à commande maître est configurée pour communiquer avec le terminal mobile intelligent par l'intermédiaire du module de communication sans fil, le terminal mobile intelligent est configuré pour stocker des programmes d'entraînement des résultats d'entraînement de rétroaction et envoyer une instruction de stimulation électrique selon les programmes ou les résultats d'entraînement, la MCU à commande maître est configurée pour commander, sur la base du signal électromyographique ou de l'instruction de stimulation reçue par le module de communication sans fil, le circuit de stimulation électrique pour émettre un courant à impulsions, et le circuit de stimulation électrique est en connexion avec les plaques d'électrode STIM par l'intermédiaire des lignes de signal et est configuré pour fournir une stimulation électrique neuromusculaire aux muscles de plancher pelvien.

2. Dispositif intelligent d'entraînement à la réadaptation des muscles du plancher pelvien selon la revendication 1, **caractérisé en ce qu'**une zone de travail d'extrémité avant (1) de l'électrode vaginale est en forme d'une goutte d'eau lisse, un intérieur de l'électrode vaginale est d'une structure de cavité, et une extrémité arrière de l'électrode vaginale est une zone d'une poignée en forme de T; les deux couples de plaques d'électrodes sont uniformément répartis dans des fenêtres correspondantes d'une paroi latérale de la cavité, une carte mère de type barre (14) est prévue au milieu de la cavité, la carte mère (14) est munie de lignes de signal, du circuit d'amplification électromyographique, du circuit d'échantillonnage AD, de la MCU à commande maître, du circuit de stimulation électrique, du module d'alimentation en énergie et du module de communication sans fil sur celle-ci, une extrémité avant de la carte mère (14) est munie d'une aiguille incurvée à simple rangée 4Pin (12), afin d'être verticalement reliée à une plaque de couvercle supérieure (2) munie d'une douille 4Pin d'adaptation (21), et la plaque de couvercle supérieure (2) est munie de 4 sondes (22) qui sont en connexion avec la douille 4Pin (21), les 4 sondes sont respectivement en contact avec et reliées aux deux couples de plaques d'électrodes ; et une carte de clavier est verticalement reliée à une extrémité de queue de la carte mère (14) et la carte de clavier est munie d'un bouton (31) sur celle-ci pour commuter en marche/arrêt le dispositif et une lampe-témoin à LED.

3. Dispositif intelligent d'entraînement à la réadaptation des muscles du plancher pelvien selon la revendication 2, **caractérisé en ce qu'**une paroi intérieure de l'électrode vaginale est munie d'une structure en forme de protubérance (13), et la plaque de couvercle supérieure (2) est fixée sur la protubérance (13) par l'intermédiaire d'une vis (23).

4. Dispositif intelligent d'entraînement à la réadaptation des muscles du plancher pelvien selon la revendication 2, **caractérisé en ce que** la poignée en forme de T (3) est munie sur celle-ci d'une zone de pression (32) correspondant au bouton (31) et d'une zone d'affichage correspondant à la lampe-témoin à LED.

5. Dispositif intelligent d'entraînement à la réadaptation des muscles du plancher pelvien selon la revendication 1, **caractérisé en ce que** le module de communication sans fil est un module Bluetooth, et le terminal mobile intelligent est un téléphone intelligent ou un ordinateur tablette.

6. Dispositif intelligent d'entraînement à la réadaptation des muscles du plancher pelvien selon la revendication 1, **caractérisé en ce qu'**un montant de chargement (4) est muni au niveau d'un emplacement où la poignée en forme de T (3) est reliée à la zone de travail (1), d'un côté intérieur du montant de chargement (4) en connexion avec une interface de chargement du module d'alimentation en énergie et d'un côté extérieur du montant de chargement (4) en connexion avec une base de chargement.
